# EUROPEAN PATENT SPECIFICATION

(11) **EP 4 501 291 B1**
(45) Date of publication and mention of the grant of the patent: **11.11.2026**
(21) Application number: 24215178.5
(22) Date of filing: 08.09.2020
(51) Int. Cl.: A01N 1/146, A61F 7/10

(54) **ORGAN CONTAINER**
ORGANBEHÄLTER
RÉCIPIENT POUR ORGANE

(30) Priority: 11.09.2019 JP 2019165110
(43) Date of publication of application: 05.02.2025
(62) Document number(s) of the earlier application(s) in accordance with Art. 76 EPC: 20194973.2 / 3 791 720
(73) Proprietor: SCREEN Holdings Co., Ltd., Kyoto-shi, Kyoto 602-8585 (JP)
(72) Inventor: TORAI, Shinji, Kyoto, 602-8585 (JP); OHARA, Masayuki, Kyoto, 602-8585 (JP)
(74) Representative: Kilian Kilian & Partner mbB

(56) References cited:
- CN-A- 105 852 982
- CN-A- 107 969 419
- CN-U- 206 851 880
- JP-A- 2019 094 315
- KOBAYASHI EIJI ET AL: "Intra-abdominal Cooling System for the Transplanted Kidney", TRANSPLANTATION DIRECT, vol. 5, no. 4, 18 March 2019 (2019-03-18), pages e438, XP093252374, ISSN: 2373-8731, DOI: 10.1097/TXD.0000000000000882

## Description

### BACKGROUND OF THE INVENTION

### Field of the Invention

The present invention relates to an organ container for accommodating an organ.

### Description of the Background Art

In organ transplant operations, an organ removed from a donor is preserved under cooled conditions. This is because if the organ is left at ordinary temperature and the bloodstream in the organ is stopped, i.e., the organ gets into a so-called warm ischemic state, the organ becomes easy to deteriorate due to metabolism in the organ. Specifically, the temperature of the organ is kept low through procedures such as pouring a low-temperature preservation solution into the isolated organ or directly spraying ice-slush saline around the organ. This suppresses organ metabolism.

However, at the time of transplanting an organ into a recipient, the organ is placed in the body cavity of the recipient and undergoes procedures such as vascular anastomosis. At this time, the organ cannot be kept under cooled conditions, so that the temperature of the organ rises due to the body temperature of the recipient or the outside air temperature and the organ gradually gets into a warm ischemic state. Hence, the surgeon carrying out the transplant operation has to maintain low-temperature conditions of the organ to be transplanted, by, for example, performing procedures such as vascular anastomosis in a time as short as possible or pouring ice or other materials into the abdominal cavity. In the latter case, not only the organ but also the fingertips of the surgeon will be cooled at the same time, and this is disadvantageous for vascular anastomosis that requires high precision.

In view of this, the inventors of the present application have proposed a technique described in Japanese Patent Application Laid-Open No. 2018-000309, in which at the time of transplanting an organ into a recipient, a sheet having a heat insulating function is inserted between the recipient and the organ to suppress a temperature rise in the organ. The sheet according to Japanese Patent Application Laid-Open No. 2018-000309 includes a heat insulating membrane and two waterproofing membranes that are face-bonded to the opposite sides of the heat insulating membrane.

However, the sheet according to Japanese Patent Application Laid-Open No. 2018-000309 does not completely cover the organ. Thus, there is a problem that a temperature rise occurs in the portion of the surface of the organ that is not covered with the sheet. Besides, end sections of the sheet inserted between the recipient and the organ may hinder the work of the surgeon.

A further prior art document is Kobayashi Eiji ET AL: "Intra-abdominal Cooling System for the Transplanted Kidney", Transplantation Direct, vol. 5, no. 4, published online 18 March 2019, page e438, XP093252374. This prior art document discloses an organ container for accommodating an organ in form of a thermo-barrier bag (TBB). The TBB comprises an expandable pouch-shaped body, which is formed of an elastomer gel, having an opening, wherein in a no-load condition a maximum width of said opening is less than a maximum width of said body, and wherein said opening is expandable to a state in which the maximum width of said opening becomes greater than the maximum width of said body in a no-load condition.

### SUMMARY OF THE INVENTION

It is an object of the present invention to provide a medical appliance that further suppresses a temperature rise in an organ and is less likely to hinder the work of a surgeon at the time of transplanting the organ into a recipient. This object is achieved by the subject matter of claim 1. Further advantageous modifications and embodiments of the invention are the subject matter of the dependent claims. Aspects of the invention are set out below.

A first aspect of the present application is an organ container for accommodating an organ, as defined in claim 1. The organ container includes, amongst others, a pouch-shaped body having contraction and expansion properties and an opening. In a no-load condition, a maximum width of the opening is less than a maximum width of the body. The opening is expandable to a state in which the maximum width of the opening becomes greater than the maximum width of the body.

According to the first aspect of the present application, the organ container covers the surface of an organ along the surface of the organ. Thus, when the organ is placed in the body cavity of the recipient, the organ container does not expand around the organ. Accordingly, the organ container is less likely to hinder work during operation. Moreover, the organ container covers most part of the organ and thereby suppresses a temperature rise in the organ.

These and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a perspective view of an organ container;
Fig. 2 is a top view of the organ container;
Fig. 3 is a sectional view of the organ container;
Fig. 4 is a diagram illustrating how an organ is accommodated in the organ container;
Fig. 5 is a diagram illustrating how the organ is accommodated in the organ container;
Fig. 6 is a diagram illustrating how the organ is accommodated in the organ container;
Fig. 7 is a flowchart of a procedure for a transplant operation using the organ container;
Fig. 8 is a sectional view of an organ container according to a variation;
Fig. 9 is a sectional view of an organ container according to another variation;
Fig. 10 is a sectional view of an organ container according to another variation; and
Fig. 11 is a sectional view of an organ container according to another variation.

### DESCRIPTION OF THE PREFERRED EMBODIMENTS

An embodiment of the present invention will be described hereinafter with reference to the drawings.

In the present application, "donors" and "recipients" may be humans, or may be non-human animals. That is, "organs" according to the present application may be human organs, or may be organs of non-human animals. The non-human animals may be rodents such as mice and rats, ungulates such as pigs, goats, and sheep, non-human primates such as chimpanzees, or other non-human mammals, or may be nonmammalian animals.

### 1. First Embodiment

### 1-1. Organ Container

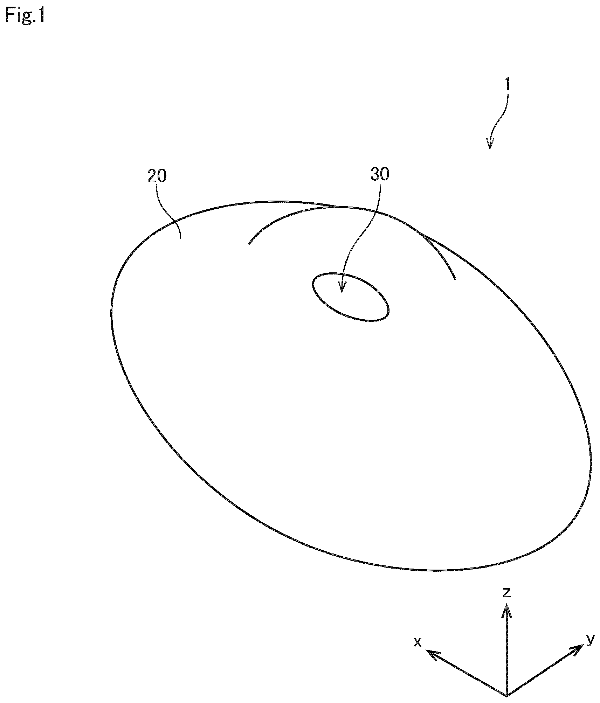

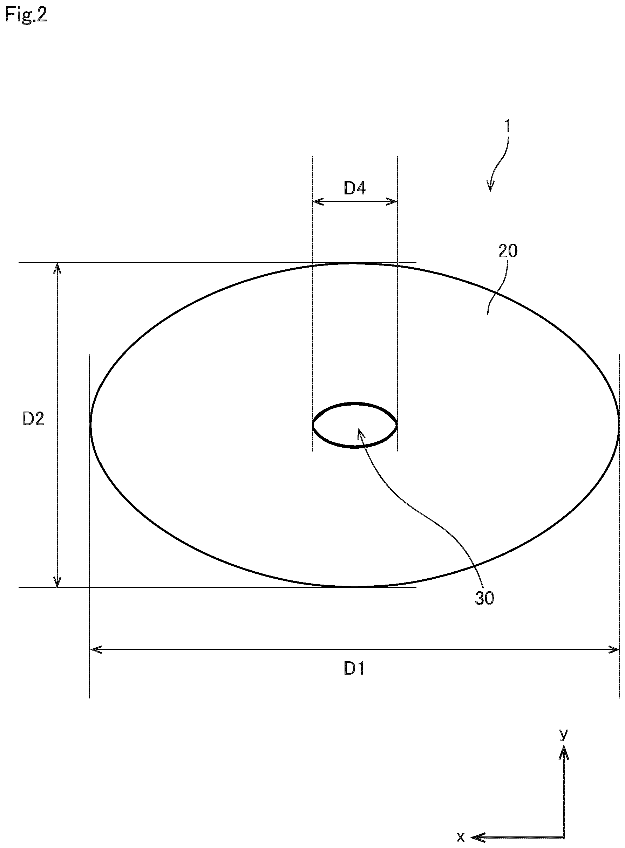

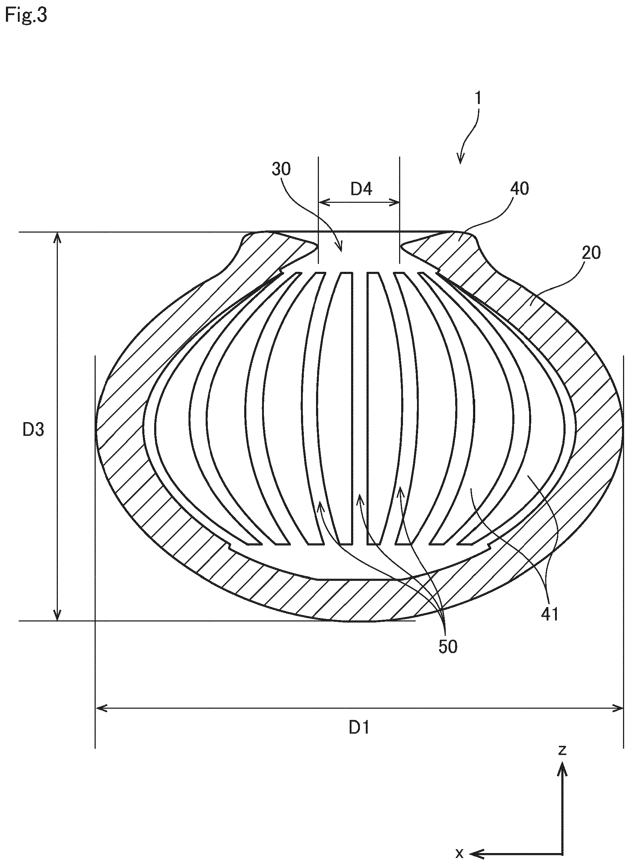

Fig. 1 is a perspective view of an organ container 1 according to a first embodiment. Fig. 2 is a top view of the organ container 1. Fig. 3 is a sectional view of the organ container 1. This organ container 1 is a container for temporarily accommodating an organ removed from a donor in a transplant operation for transplanting the organ into the recipient. That is, the organ container 1 is a medical appliance for use in organ transplant operations.

Examples of the organ that can be accommodated in the organ container 1 include a kidney, a heart, and a lung. Blood vessels of these organs that are to be anastomosed in transplant operations are concentrated on one side of the organs. The structure of the organ container 1 according to the present embodiment is particularly suitable for these organs. However, the organ container according to the present invention may be configured to accommodate other organs such as a liver.

In the following description, the up-down direction is defined such that the side of the organ container 1 with an opening 30 is considered as the upper side and the bottom side of the organ container 1 opposite to the opening 30 is considered as the lower side. In the following description, the up-down direction is also referred to as a z direction, a direction along the long sides of the organ container 1 when viewed in the z direction is referred to as an x direction, and a direction along the short sides of the organ container 1 when viewed in the z direction is referred to as a y direction, as illustrated in Fig. 1. Note that the definition of the up-down direction does not intend to limit the orientation of the organ container 1 during use.

As illustrated in Fig. 1, the organ container 1 includes a pouch-shaped body 20 with contraction and expansion properties. The body 20 has an opening 30 and a thick part 40. The opening 30 communicates between the inside of the body 20 and an exterior space. The thick part 40 surrounds the opening 30 in a ring shape.

The organ container 1 according to the present embodiment as a whole is generally an ellipsoid. That is, the body 20 has generally an ellipsoidal shape. This organ container 1 is, in particular, configured to accommodate a kidney. The organ container 1 as a whole is made in generally an ellipsoidal shape so that the inner surface of the body 20 has a shape that easily fits along the surface of a kidney.

To describe the relation of dimensions, in the following description, a length of the body 20 in the direction along the long sides (x direction) is referred to as a length D1, a length of the body 20 in the direction along the short sides (y direction) is referred to as a length D2, and a length of the body 20 in the up-down direction (z direction) is referred to as a length D3, as illustrated in Figs. 2 and 3.

The body 20 is formed of an elastomer gel. Specifically, the body 20 is formed of a thermoplastic elastomer, an urethane elastomer, or an oil bleeding silicone gel. The body 20 has a hardness of, for example, E10 to A10 according to a durometer hardness test that complies with Japanese Industrial Standards JIS K 6253-3:2012. An elongation percentage at break for the body 20 is higher than or equal to 1000%.

Forming the body 20 of such a material makes the body 20 and the opening 30 easy to expand and less likely to break. Forming the body 20 of such a material also makes it possible to reduce the possibility that the temperature outside the body 20 propagates to the organ in the body 20 at the time of accommodating the organ in the body 20.

The opening 30 is an opening for inserting an organ into the internal space of the body 20. The opening 30 also plays a role as a connection port for connecting blood vessels or other pathways connected to the organ to the outside while the organ is accommodated in the body 20.

In the present embodiment, the opening 30 has an elliptical shape. In a no-load condition in which no artificial loads are imposed on the organ container 1, a length of the opening 30 along the major axis is referred to as a length D4. The length D4, which is a maximum width of the opening 30 in a no-load condition, is shorter than the length D1, which is a maximum width of the body 20.

The thick part 40 is a partial area that is greater in thickness than the surrounding area. With increasing thickness, the elastomer gel, which forms the body 20, has a higher capability to contract in a direction returning to its original state against loads applied in the direction of expansion. Thus, surrounding the opening 30 in a ring shape by the thick part 40 makes it possible to increase an elastically deformable amount of the area around the opening 30 and to improve strength around the opening 30. That is, it is possible, at the time of expanding the opening 30, to suppress plastic deformation around the opening 30 and breakage of the opening 30.

Besides, at the time of accommodating the organ inside, the thick part 40 comes in intimate contact with the organ. This suppresses a protrusion of the organ from the opening 30 and suppresses an outflow of a liquid held between the organ and the body 20 from the opening 30.

The inner surface of the body 20 has an uneven shape. Specifically, as illustrated in Fig. 3, the inner surface of the body 20 has a plurality of projections 41 that project toward the internal space of the body 20. The projections 41 extend in the up-down direction from the opening 30 toward the bottom of the body 20. Accordingly, a groove 50 is formed between each pair of adjacent projections 41. That is, the inner surface of the body 20 has a plurality of grooves 50 extending in the up-down direction.

This uneven inner surface of the body 20 allows a preservation solution for cooling to be held in the interstices between the organ and depressions in the inner surface of the body 20 when the organ is accommodated in the body 20. In particular, in the case where the inner surface of the body 20 has a plurality of grooves 50 extending in the up-down direction as in the present embodiment, the preservation solution for cooling poured inside from the opening 30 can easily reach the bottom side. That is, the preservation solution for cooling, held between the organ and the inner surface of the body 20, is likely to spread across the inner surface.

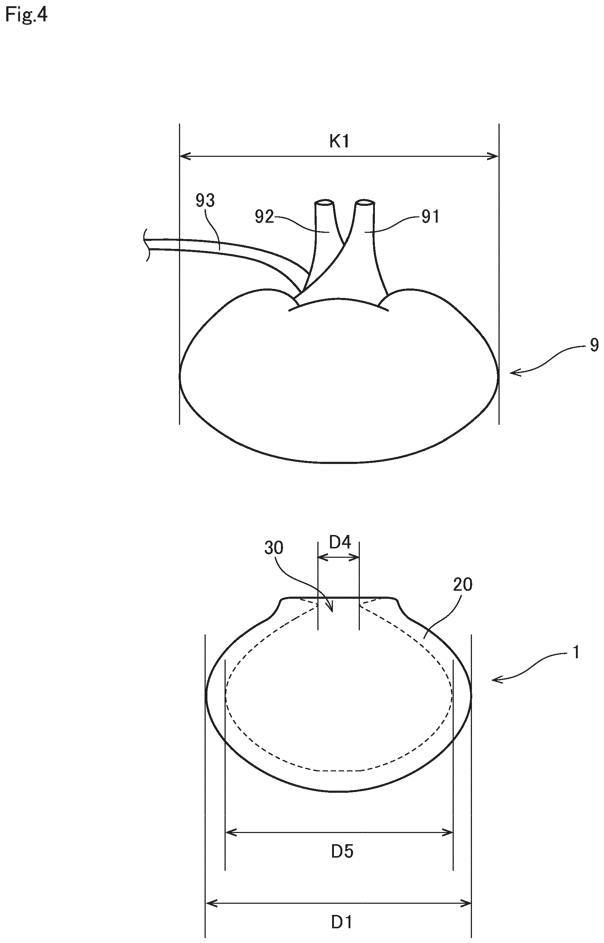

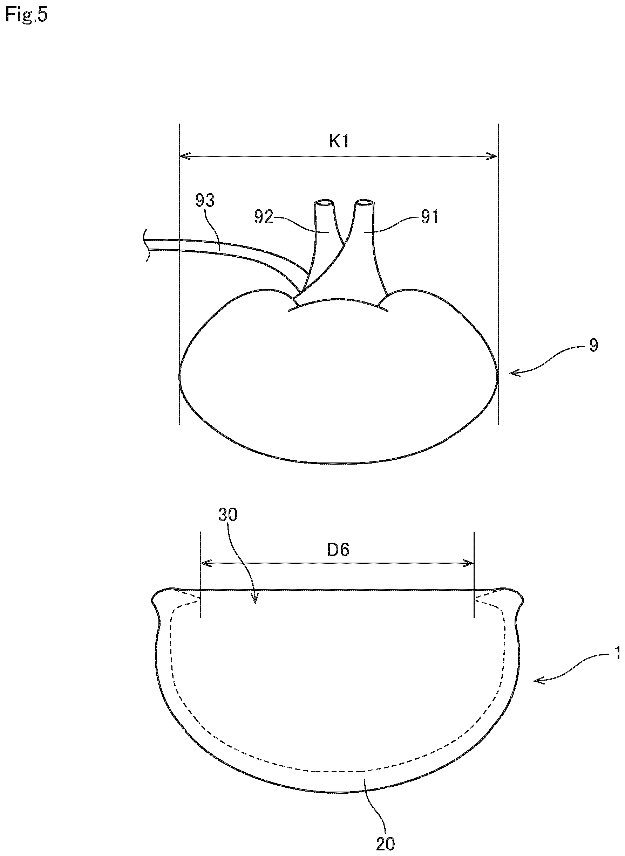

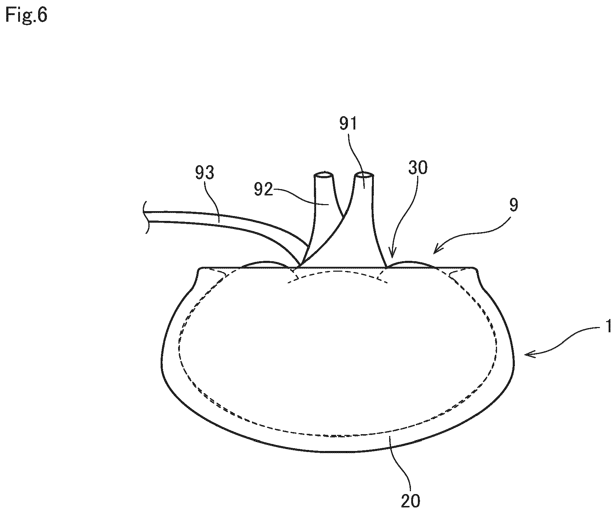

Figs. 4 to 6 are diagrams illustrating how a kidney 9, which is one example of the organ, is accommodated in the organ container 1. Specifically, Fig. 4 is a diagram illustrating the kidney 9 and the organ container 1 that is in a no-load condition side by side. Fig. 5 is a diagram illustrating that the opening 30 of the organ container 1 is expanded in order to accommodate the kidney 9. Fig. 6 is a diagram illustrating that the kidney 9 is accommodated in the organ container 1.

As illustrated in Fig. 4, in a no-load condition, the length D1, which is the maximum width of the body 20 of the organ container 1, is shorter than a maximum width of the kidney 9, i.e., a length K1. That is, in a no-load condition, a maximum width of the inner surface of the body 20, i.e., a length D5, is shorter than the length K1, which is the maximum width of the kidney 9. Thus, the length D4, which is the maximum width of the opening 30 in a no-load condition, is shorter than the length K1, which is the maximum width of the kidney 9.

The opening 30 is expandable to a state in which the maximum width of the opening 30 becomes greater than the length D1, which is the maximum width of the body 20 in a no-load condition. Thus, the kidney 9 can be easily accommodated in the body 20. In Fig. 5, a state is illustrated in which the opening 30 is expanded until the maximum width of the opening 30 becomes a length D6 longer than the length D1. In this way, the operator expands the opening 30 to accommodate the kidney 9 in the body 20 through the opening 30. In the organ container 1 according to the present embodiment, the opening 30 can be expanded to a state in which the maximum width of the opening 30 becomes greater than the length K1, which is the maximum width of the kidney 9. Therefore, the kidney 9 can be more easily accommodated in the body 20.

Then, when the kidney 9 is accommodated in the body 20 as illustrated in Fig. 6, the entire inner surface of the body 20 including the periphery of the opening 30 fits along the surface of the kidney 9. As described above, in a no-load condition, the inner surface of the body 20 is smaller than the outer surface of the kidney 9. Thus, when the kidney 9 is accommodated in the organ container 1, the inner surface of the body 20 fits along the outer surface of the kidney 9, and the projections 41 on the inner surface of the body 20 come in intimate contact with the outer surface of the kidney 9. Accordingly, the kidney 9 is appropriately held without being moved inside the body 20. As a result, it is possible to suppress damage to the kidney 9.

### 1-2. Procedure for Organ Transplantation

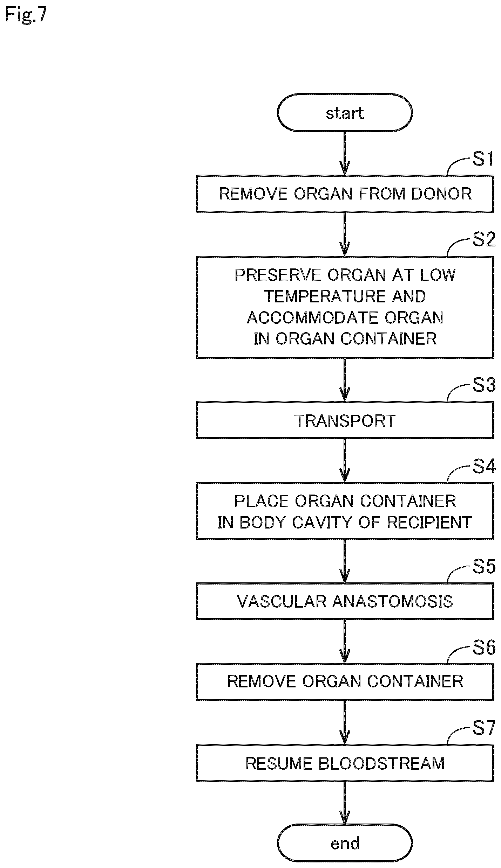

Next, a procedure for a transplant operation using the above-described organ container 1 will be described. Fig. 7 is a flowchart illustrating the procedure for the transplant operation using the organ container 1. The following description is given of the case where the kidney 9 is to be transplanted using the organ container 1.

In the transplant operation, first the kidney 9 is removed from a donor (step S1). Specifically, blood vessels 91 and 92 and an ureter 93 that extend from the kidney 9 of the donor (see Figs. 4 to 6) are cut, and the kidney 9 is taken out of the body cavity of the donor.

The removed kidney 9 is preserved while being immersed in a low-temperature preservation solution. The kidney 9 is also accommodated in the organ container 1 (step S2). For example, saline kept at 4°C is used as the preservation solution. If the organ is left at ordinary temperature and the bloodstream in the organ is stopped, i.e., the organ gets into a so-called warm ischemic state, the organ becomes easy to deteriorate due to metabolism in the organ. Thus, in step S2, the kidney 9 is preserved at a temperature lower than ordinary temperature so as to suppress deterioration of the kidney 9.

Alternatively, in step S2, tubes may be connected to the blood vessels 91 and 92 of the kidney 9, and the kidney 9 may be preserved while being perfused with a preservation solution. Note that the perfusion using the preservation solution may continue until step S4 described later.

The timing when the kidney 9 is accommodated in the organ container 1 may be before the kidney 9 is immersed in the preservation solution, or may be after the kidney 9 is immersed in the preservation solution for a while and sufficiently cooled. When the kidney 9 is accommodated in the organ container 1, the opening 30 of the organ container 1 is opened, and the kidney 9 is inserted through the opening 30 into the body 20 as illustrated in Fig. 5. Accordingly, the kidney 9 is held inside the pouch-shaped body 20 as illustrated in Fig. 6. At this time, a low-temperature preservation solution is poured into the space between the kidney 9 and the inner surface of the body 20, using a syringe or a pipette. Also, the kidney 9 embraced by the organ container 1 is immersed again in a low-temperature preservation solution to maintain a low-temperature preservation condition.

The kidney 9 held by the organ container 1 is transported from the donor side to the recipient side while being immersed in the low-temperature preservation solution (step S3). The kidney 9 transported to the recipient side continues to be held by the organ container 1 and to be immersed in the low-temperature preservation solution until just before transplantation.

Then, the abdomen of the recipient is opened, and the organ container 1 accommodating the kidney 9 therein is placed in the body cavity of the recipient (step S4). Then, the blood vessels of the recipient are anastomosed to the blood vessels 91 and 92 of the kidney 9 exposed to the outside through the opening 30 of the organ container 1 (step S5). In the case where the organ to be transplanted is the kidney 9, the ureter 93 is also connected to the urinary bladder.

During the work in steps S4 and S5, the kidney 9 accommodated in the organ container 1 is placed within the body cavity. At this time, since the organ container 1 has a thermal insulating function, it is possible to suppress a temperature rise in the kidney 9 due to the body temperature of the recipient or the outside air temperature. This reduces the possibility that the kidney 9 gets into a warm ischemic state and its deterioration progresses due to metabolism. As a result, it is possible to suppress the occurrence of troubles after operation.

In steps S4 and S5, a low-temperature preservation solution is poured at regular time intervals into the organ container 1, using a syringe or a pipette. For example, a low-temperature preservation solution is poured every few minutes into the organ container 1. This further suppresses a temperature rise in the kidney 9.

Thereafter, the opening 30 of the organ container 1 is opened, and the kidney 9 that has undergone vascular anastomosis is taken out of the organ container 1. Then, the organ container 1 is removed from the body cavity of the recipient (step S6). Thereafter, the bloodstream from the anastomosed blood vessels of the recipient to the kidney 9 is resumed (step S7).

This organ container 1 covers the surface of the kidney 9 along the surface of the kidney 9. Thus, part of the organ container 1 does not expand around the kidney 9. For example, end sections or the like of the organ container 1 do not expand around the kidney 9. Accordingly, the organ container 1 is less likely to hinder work during operation. Moreover, the organ container 1 covers most part of the surface of the kidney 9 and thereby further suppresses a temperature rise in the kidney 9 and prevents damage to the surface of the kidney 9 during operation.

This organ container 1 is formed of a raw material having elasticity. Thus, the organ container 1 can absorb impacts on the kidney 9 during transport or during operation. Accordingly, it is possible to suppress damage to the kidney 9.

### 2. Variations

While a principal embodiment of the present invention has been described thus far, the present invention is not intended to be limited to the embodiment described above.

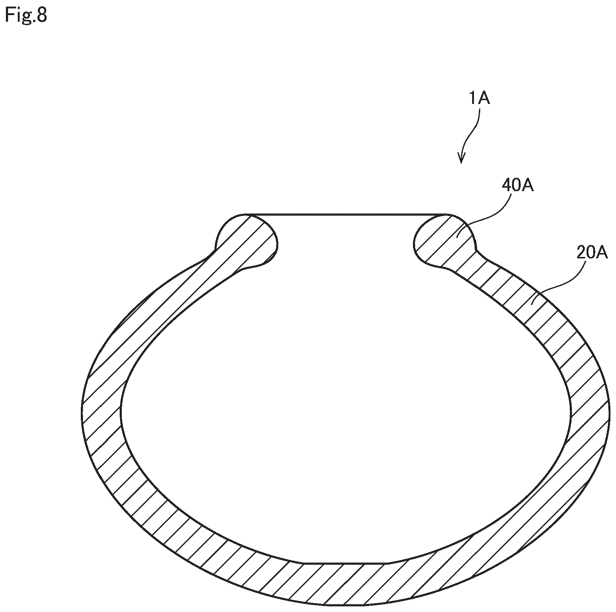

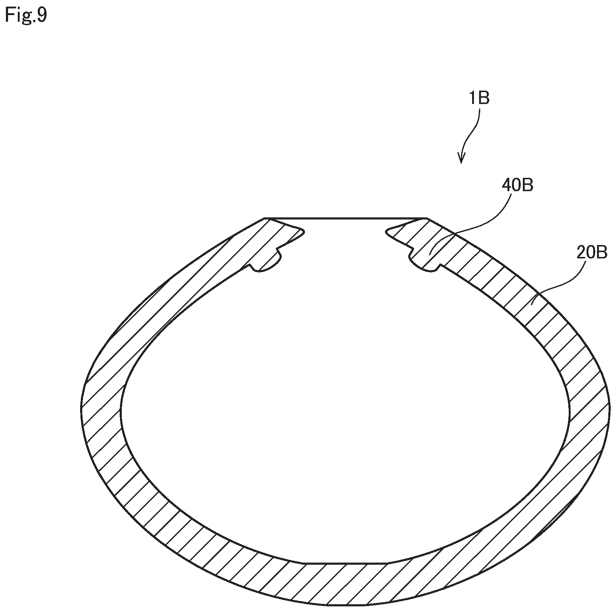

Fig. 8 is a sectional view of an organ container 1A according to a variation. Fig. 9 is a sectional view of an organ container 1B according to another variation. In Figs. 8 and 9, uneven shapes of the inner surfaces of bodies 20A and 20B are not illustrated.

In the organ container 1 according to the above-described embodiment, the thick part 40 is greater in thickness than the surrounding area as a result of protruding toward the outer surface of the body 20 without protruding toward the inner surface of the body 20. In contrast, in the organ container 1A in the example illustrated in Fig. 8, a thick part 40A has a generally circular sectional shape. Thus, the thick part 40A protrudes toward both of the inner and outer surfaces of the body 20A. In the organ container 1B in the example illustrated in Fig. 9, a thick part 40B protrudes toward the inner surface of the body 20B without protruding toward the outer surface of the body 20B.

As in the examples in Figs. 8 and 9, the thick part may protrude toward both of the inner and outer surfaces of the body. The shape of the thick part may be appropriately changed.

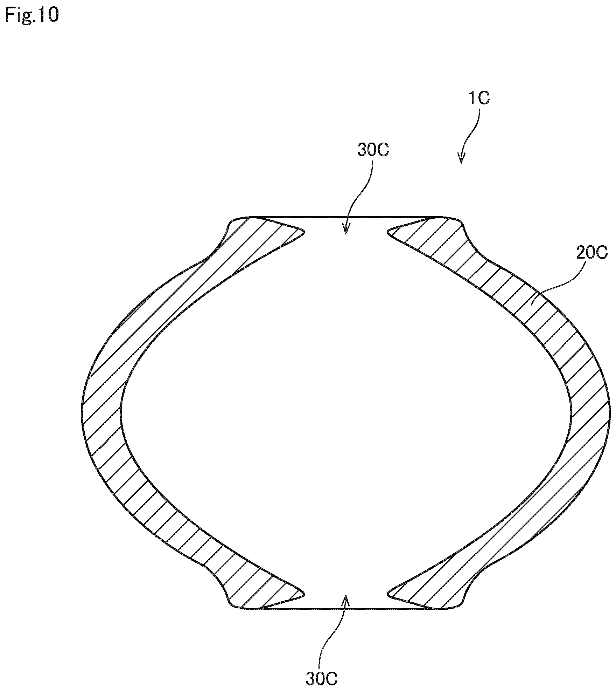

Fig. 10 is a sectional view of an organ container 1C according to another variation. As in Figs. 8 and 9, an uneven shape of the inner surface of the body 20C is not illustrated in Fig. 10. The organ container 1 according to the above-described embodiment includes one opening 30 and is thus suitable for organs such as a kidney, a heart, or a lung in which blood vessels to be anastomosed during transplant operations are concentrated on one side of the organs.

In contrast, a body 20C of the organ container 1C in the example illustrated in Fig. 10 has two openings 30C. Thus, the organ container 1C is suitable for organs such as a liver, a pancreas, or a spleen in which blood vessels to be anastomosed during transplant operations extend in multiple directions. As described above, the number of openings in the body is not limited to one. In the case where the body has a plurality of openings, these openings do not necessarily have to be of the same size. An opening through which a blood vessel passes may be smaller than an opening for accommodating an organ.

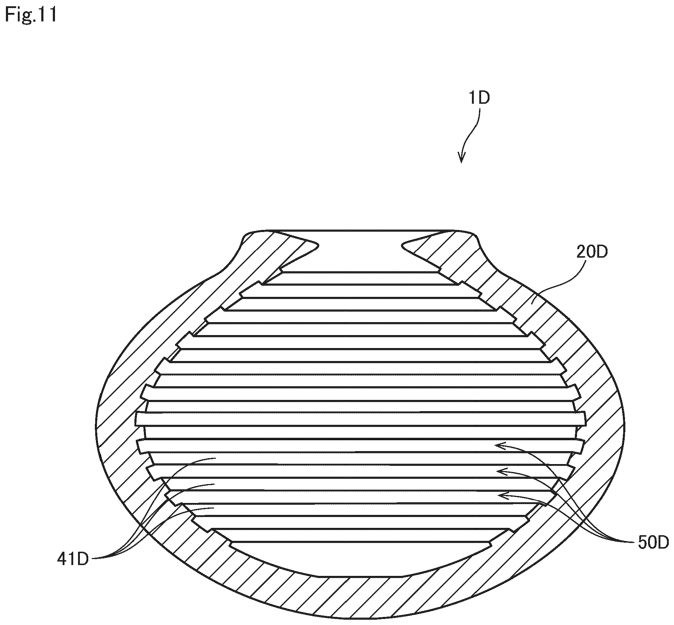

Fig. 11 is a sectional view of an organ container 1D according to another variation. In the organ container 1D illustrated in Fig. 11, the inner surface of a body 20D has a plurality of projections 41D that extend in a direction orthogonal to the up-down direction. Accordingly, a groove 50D is formed between each pair of adjacent projections 41D. That is, the inner surface of the body 20D has a plurality of grooves 50D extending in the direction orthogonal to the up-down direction.

In the above-described embodiment, the grooves formed in the inner surface of the body extend in the up-down direction, but the present invention is not limited thereto. The direction of extension of the grooves may be a direction orthogonal to the up-down direction as in the example illustrated in Fig. 11.

Alternatively, the grooves may be formed in, for example, a spiral shape in the inner surface of the body. As another alternative, a configuration is also possible in which the grooves include a plurality of first grooves that extend generally parallel to one another in a predetermined direction, and a plurality of second grooves that extend generally parallel to one another in a predetermined direction different from the direction of extension of the first grooves, and at least some of the first grooves and at least some of the second grooves intersect with one another in lattice form. The uneven shape of the inner surface of the body is not limited to projections and grooves, both extending in a predetermined direction. For example, the inner surface of the body may have projections or depressions of indefinite shape that are arranged at random intervals.

In the above-described embodiment, the body 20 of the organ container 1 is formed of a single type of material, but the present invention is not limited thereto. For example, the body 20 may be configured of a plurality of layers formed of different materials. For example, a layer that is close to the outer surface of the body may have a greater durometer hardness value than a layer that forms the inner surface of the body that comes in contact with an organ. Also, a coating may be applied to the outer surface of the body in order to prevent external damage.

A detailed structure of the organ container does not necessarily have to match completely with the structure illustrated in each drawing of the present application. Each component given in the embodiment and variations described above may be combined appropriately within a range that causes no contradictions.

While the invention has been shown and described in detail, the foregoing description is in all aspects illustrative and not restrictive. It is therefore understood that numerous modifications and variations can be devised without departing from the scope of the invention as long as covered by the claims.

## Claims

1. An organ container (1) for accommodating an organ, comprising:
an expandable pouch-shaped body (20), which is formed of an elastomer gel, having an opening (30),
wherein in a no-load condition, a maximum width of said opening (30) is less than a maximum width of said body (20), wherein
said opening (30) has the capability to expand and contract to a state in which the maximum width of said opening (30) becomes greater than the maximum width of said body (20) in a no-load condition,
said body (20) has a ring-shaped thick part (40) that surrounds said opening (30), and
said thick part (40) is a partial area that is greater in thickness than the surrounding area such that said thick part (40) is adapted to increase an elastically deformable amount of the area around the opening (30).

2. The organ container (1) according to claim 1, wherein
an inner surface of the body (20, 20D) has a plurality of projections (41, 41D), which project toward the internal space of the body, and a plurality of grooves (50, 50D), each groove being formed between a pair of adjacent projections.

3. The organ container (1) according to claim 2, wherein
said plurality of grooves (50) includes:
a plurality of first grooves; and
a plurality of second grooves, each intersecting with at least some of said first grooves.

4. The organ container (1) according to any one of claims 1 to 3, wherein
said body (20) has generally an ellipsoidal shape.

5. The organ container (1) according to any one of claims 1 to 4, wherein
said body (20) has a hardness of E10 to A10 according to a durometer hardness test that complies with Japanese Industrial Standards JIS K 6253-3:2012.

6. The organ container (1) according to any one of claims 1 to 5, wherein
said body (20) is formed of a thermoplastic elastomer, an urethane elastomer, or an oil bleeding silicone gel.

## Patentansprüche

1. Organbehälter (1) zum Aufnehmen eines Organs, umfassend:
einen expandierbaren beutelförmigen Körper (20), der aus einem Elastomergel ausgebildet ist und eine Öffnung (30) aufweist,
wobei in einem unbelasteten Zustand eine maximale Breite der Öffnung (30) geringer ist als eine maximale Breite des Körpers (20), wobei
die Öffnung (30) die Fähigkeit aufweist, sich zu einem Zustand auszudehnen und zusammenzuziehen, in dem die maximale Breite der Öffnung (30) größer als die maximale Breite des Körpers (20) in einem unbelasteten Zustand wird,
der Körper (20) einen ringförmigen verdickten Abschnitt (40) aufweist, der die Öffnung (30) umgibt, und
der verdickte Abschnitt (40) ein Teilbereich ist, der eine größere Dicke als der umgebende Bereich aufweist, so dass der verdickte Abschnitt (40) dazu eingerichtet ist, einen elastisch verformbaren Betrag des Bereichs um die Öffnung (30) zu erhöhen.

2. Organbehälter (1) nach Anspruch 1, wobei
eine Innenfläche des Körpers (20, 20D) eine Vielzahl von Vorsprüngen (41, 41D), die in Richtung eines Innenraums des Körpers vorstehen, und eine Vielzahl von Nuten (50, 50D) aufweist, wobei jede Nut zwischen einem Paar benachbarter Vorsprünge ausgebildet ist.

3. Organbehälter (1) nach Anspruch 2, wobei
die Vielzahl von Nuten (50) umfasst:
eine Vielzahl von ersten Nuten; und
eine Vielzahl von zweiten Nuten, die jeweils zumindest einige der ersten Nuten schneiden.

4. Organbehälter (1) nach einem der Ansprüche 1 bis 3, wobei
der Körper (20) eine im Wesentlichen ellipsoidförmige Gestalt aufweist.

5. Organbehälter (1) nach einem der Ansprüche 1 bis 4, wobei
der Körper (20) eine Härte von E10 bis A10 gemäß einer Durometer-Härteprüfung aufweist, die der japanischen Industrienorm JIS K 6253-3:2012 entspricht.

6. Organbehälter (1) nach einem der Ansprüche 1 bis 5, wobei
der Körper (20) aus einem thermoplastischen Elastomer, einem Urethanelastomer oder einem ölhaltigen Silikongel gebildet ist.

## Revendications

1. Récipient pour organe (1) destiné à accueillir un organe, comprenant :
un corps extensible en forme de poche (20), qui est formé d'un gel élastomère, ayant une ouverture (30),
dans lequel, à l'état sans charge, la largeur maximale de ladite ouverture (30) est inférieure à la largeur maximale dudit corps (20), dans lequel
ladite ouverture (30) a la capacité de se dilater et de se contracter jusqu'à un état dans lequel la largeur maximale de ladite ouverture (30) devient supérieure à la largeur maximale dudit corps (20) à l'état sans charge,
ledit corps (20) a une partie épaisse en forme d'anneau (40) qui entoure ladite ouverture (30), et
ladite partie épaisse (40) est une zone partielle dont l'épaisseur est supérieure à celle de la zone environnante, de sorte que ladite partie épaisse (40) soit adaptée pour augmenter une quantité élastiquement déformable de la zone autour de l'ouverture (30).

2. Récipient pour organe (1) selon la revendication 1, dans lequel
une surface intérieure du corps (20, 20D) a une pluralité de projections (41, 41D), qui se projettent vers l'espace interne du corps, et une pluralité de rainures (50, 50D), chaque rainure étant formée entre une paire de projections adjacentes.

3. Récipient pour organe (1) selon la revendication 2, dans lequel
ladite pluralité de rainures (50) comprend :
une pluralité de premières rainures ; et
une pluralité de secondes rainures, chacune intersectant au moins certaines desdites premières rainures.

4. Récipient pour organe (1) selon l'une quelconque des revendications 1 à 3, dans lequel
ledit corps (20) présente globalement une forme ellipsoïdale.

5. Récipient pour organe (1) selon l'une quelconque des revendications 1 à 4, dans lequel
ledit corps (20) a une dureté de E10 à A10 selon un test de dureté au duromètre qui est conforme aux Normes Industrielles Japonaises JIS K 6253-3:2012.

6. Récipient pour organe (1) selon l'une quelconque des revendications 1 à 5, dans lequel
ledit corps (20) est formé d'un élastomère thermoplastique, d'un élastomère d'uréthane ou d'un gel de silicone à exsudation d'huile.
